# EUROPEAN PATENT SPECIFICATION

(11) **EP 0 495 842 B1**
(45) Date of publication and mention of the grant of the patent: **13.12.1995**
(21) Application number: 90915146.6
(22) Date of filing: 09.10.1990
(51) Int. Cl.: A61F 2/42

(54) **PROSTHETIC FINGER JOINT**
FINGERGELENKPROTHESE
ARTICULATION DE DOIGT PROTHETIQUE

(30) Priority: 09.10.1989 GB 8922697
(43) Date of publication of application: 29.07.1992
(73) Proprietor: NEOLIGAMENTS LIMITED, Leeds LS2 9HD (GB)
(72) Inventor: SEEDHOM, Bahaa, Botros, Leeds LS2 9HD (GB); HARRISS, Julian, Vancouver, British Columbia (CA); COLLINS, Simon, Leeds LS2 9HD (GB)
(74) Representative: Orr, William McLean
(86) International application number: GB9001553
(87) International publication number: WO9104718

(56) References cited:
- EP-A- 0 278 184
- WO-A-89/09580
- US-A- 4 304 011
- US-A- 4 352 212

## Description

This invention relates to a prosthetic finger joint and is particularly, though not exclusively, concerned with a replacement metacarpophalangeal (MCP) joint.

It is known to provide prosthetic finger joints which comprise a stem having a ball end, in which the stem is secured to a centre region of a planar face of the ball end and projects substantially perpendicularly therefrom.

To replace a damaged or deteriorated finger joint between two finger sections, or a MCP joint, it is necessary to remove the remnants of the natural material providing the pivot axis between the finger sections and to drill a passage in one of the finger sections into which the stem is introduced.

The ball ends of existing prosthetic finger joints are generally hemispherical, and the spherical outer face provides a bearing surface which permits pivoting of the other finger section relative thereto. However, given that the ball end is hemispherical and has its planar end face extending perpendicular to the stem, this limits the extent of relative pivotal movement which can take place.

The most popular MCP replacement is the Swanson silicone rubber prosthesis which is a one component prosthesis with two stems introduced into the bone cavities, without any fixation. It restores the joint from a cosmetic point of view, but not its function to any appreciable extent. Also, in the course of time, natural ligament material may be completely damaged by disease. Absence of ligaments renders a joint unstable.

Furthermore, in the Swanson prosthesis, with each flexing of the joint, there will be a tendency for the stems to move longitudinally in their respective bone cavities, and this causes abrasion of the walls Of the cavities which reduces the retention of the stems in position over a period of time, and also is undesirable in wearing away natural bone material which creates dust within the bone cavities.

It has been proposed in WO89/09580 in the name of B B Seedhom to provide a prosthetic finger joint for implantation between two finger bone sections and which comprises a stem for introduction into a passage formed in a first of the finger bone sections, and a part spherical bearing provided at one end of the stem to permit pivoting of a second finger bone section relative to the first finger bone section, the bearing being arranged to seat on an inclined end face of the first finger section so as to be non-perpendicular inclined to the general axis of the passage, and in which:
the part spherical bearing has a substantially planar seating face which is engageable with said inclined end face of the first finger section, and said stem projects non-perpendicularly from said planar seating face to be receivable by said passage.

The present invention has been developed with a view to improve this prior proposal in regard to the construction of the stem, and also the manner by which it can be coupled with the head, in order to provide for an improved method of implantation of the stem into its bone passage which is easy to carry out and is reliable, but which enables the head to be coupled with the stem as a separate component and subsequent to implantation of the stem.

It is also known from EP-A-0278184 to provide a prosthetic finger joint for implantation between two finger bone sections and which comprises an implantable stem portion for introduction into a passage formed in a first of the finger bone sections, and a head portion which is engageable with an end face of the first finger section and which serves to permit pivoting of a second of the finger bone sections relative to the first finger bone section. Further, the stem portion and the head portion are formed as separate components which are capable of being coupled together after implantation of the stem portion, and the stem portion is shaped externally so as to be capable of being implanted in its respective bone passage and to be anchorable therein. Also, the head portion and the stem portion are provided with interfitting components which enable the head portion to be slidably assembled with the stem portion after implantation of the stem portion in its bone passage, and these interfitting components comprise an elongate projection provided on one of the portions and a socket in the other of the portions to receive the projection for axial slidable movement therein.

According to the invention, a prosthetic finger joint of the type known from EP-A-0278184 is characterised in that the head portion comprises a hemispherical head having a substantially planar seating face from which said projection extends non-perpendicularly.

In one embodiment, the projection is axially slidable in the socket and is non-rotatable relative thereto.

In a further embodiment, the projection is axially slidable in the socket and in such a way that relative rotation between the projection and the socket is permitted. The stem portion may therefore comprise a sleeve having a passage therein which forms said socket.

The prosthetic joint may form an unconnected joint, and includes an implantable socket device receivable by the second bone section and slidably engageable with the hemispherical head to permit relative pivoting between the first and second bone sections.

The prosthetic joint may comprise a connected joint, in which case it may include an implantable component receivable by the second bone section and co-operable with the head portion to form the connected joint.

In the connected joint, the implantable component may be connected to the head portion via a hinged connection. This connection may comprise a yoke and a tongue pivotally mounted between the limbs of the yoke.

The yoke may be joined to the head portion, and the tongue is joined to the implantable component.

In an alternative arrangement, the yoke is joined to the implantable component and the tongue is joined to the head portion.

Preferably, the hinged connection is a loose connection to permit any required degree of abduction / adduction movement.

The components of the prosthetic joint may be made of any suitable implantable material, and comprising suitable plastics material or suitable metal.

To permit variation in the depth of insertion of the projection into the socket, and thereby to vary the tension of the joint, a set of spacer washers may be provided, positionable on the projection adjacent to the head portion thereby to vary the extent of insertion of the projection.

To provide a non-rotative coupling between the head portion and the stem portion, it is preferred that the socket has a multi-sided profile and the projection has a corresponding profile. By way of example, the profile may be hexagonal, and this will then permit adjustment of the head portion between six different angular positions relative to the axis of the stem portion, to provide the best possible seating of the head portion on the inclined end face of the first finger bone section.

Preferably, the projection and socket have slidable interengagement, which can permit relative longitudinal displacement to take place between the projection and the socket, which may be desirable to facilitate flexing of the first and second finger bone sections. If such longitudinal displacement does take place, this will be by sliding interengagement between the components of the prosthesis, and therefore without any risk of damage being done to the natural bone material.

Embodiments of prosthetic finger joint according to the invention will now be described in detail, by way of example only, with reference to the accompanying drawings, in which:
Figure 1 is a diagrammatic side view of a first embodiment of prosthetic finger joint, which is intended to be used as an implant to replace an existing MCP joint;
Figure 2 is a view in the direction of the arrow X in Figure 1 of a planar end face of a hemispherical head component of the prosthetic joint shown in Figure 1;
Figure 3 is a schematic perspective view of a separate stem component of the prosthetic joint shown in Figure 1;
Figure 4 is a schematic side view, showing in more detail, the construction of an implantable socket device of the prosthetic device shown in Figure 1;
Figure 5 is a view, similar to Figure 4, of an alternative construction of the implantable socket device;
Figure 6a shows an end view of an externally screw threaded portion of the devices shown in Figures 4 and 5, to illustrate a hexagonal socket formed in one end thereof, by means of which the device can be screwed into position by the use of an Allen key or the like;
Figure 6b is a similar view of an alternative means for use in rotating the screw threaded member;
Figure 7 is an exploded view of a prosthetic finger joint, generally similar to the embodiment shown in Figure 1;
Figure 7a is a view, similar to Figure 7, of a modified arrangement of stem-receiving sleeve;
Figure 8 is a detail view of a further arrangement of stem-receiving sleeve;
Figure 9 is an exploded view of a further embodiment in the form of a connected prosthetic finger joint;
Figure 10 is a plan view of part of the embodiment shown in Figure 9;
Figure 11 is a side view of a further arrangement of connected prosthetic finger joint;
Figure 12 is a plan view corresponding to Figure 11; and,
Figure 13 is a side view, to an enlarged scale, of an unconnected prosthetic finger joint and provided with spacer washers to vary the depth of insertion of the stem into the stem-receiving sleeve.

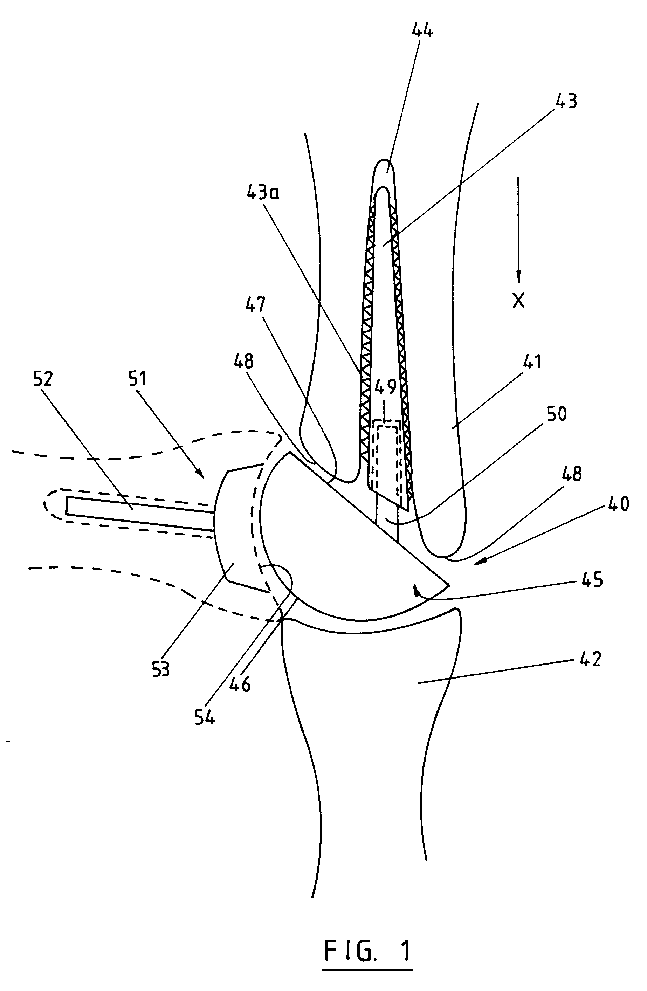

Referring now to Figure 1 of the drawings, a prosthetic finger joint according to the invention is designated generally by reference 40, and is intended for implantation between a first finger bone section 41 and a second finger bone section 42, which may be the finger bone sections adjacent to the MCP joint. The finger joint 40 comprises a stem 43 which is adapted for introduction into a passage 44 formed in the first section 41, and a part spherical bearing head 45 which has a hemispherical bearing surface 46 over which the finger section 42 can pivot through approximately 90° from the position shown in full lines in Figure 1 to the position shown in dashed outline. The head 45 also has a planar seating face 47 (which may be a generally annular surface) which engages an inclined end face 48 of the first finger section 41 after necessary implantation surgery.

It will be noted from the side illustration of Figure 1 that the seating face 47 is non-perpendicularly inclined to the general axis of passage 44, and that the stem 43 projects non-perpendicularly from the seating face 47 when it is received by the passage 44 and the head 45 is coupled therewith, in a manner described in more detail below.

The stem 43 and the head 45 are formed as separate components which are capable of being coupled together via a non-rotative coupling after implantation of the stem 43 in passage 44. To achieve implantation and anchoring of the stem 43 in its bone passage 44, the stem 43 is externally threaded so that it can become anchored in a required position within the bone passage by simple rotation of the stem. However, more preferred means of implantation comprise simple push-fit type assemblies as described below in respect of further embodiments. However, common to all of the embodiments of the invention will be the fact that each "stem portion" (a stem-forming and / or stem-receiving sleeve) is externally shaped so as to be capable of being implanted in its bone passage and to be anchorable therein e.g. by simple push or press-fit and / or accompanied by rotation to cause improved gripping engagement of any suitable external formation to engage the inner wall of the bone passage.

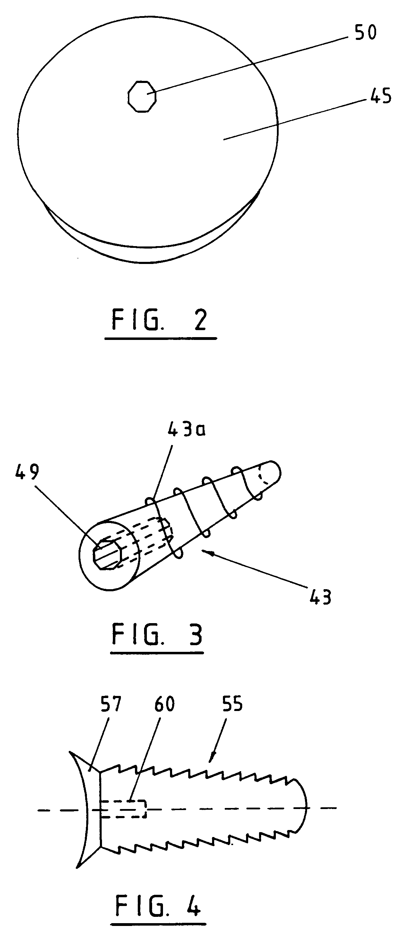

In the illustrated embodiment, as shown in Figures 1 and 3, the stem 43 is tapered, and the bone passage 44 formed in finger bone section 41 has a similar tapering section. This therefore enables the stem 43 to be introduced a long way into the passage 44 by a simple pushing action, which can therefore be carried out quite quickly, and then anchoring takes place by simple turning action applied to the stem 43, which might be less than a full turn, or perhaps a full turn or a little more, to achieve the required degree of biting engagement of the external thread 43a of the stem 43 with the internal wall of the passage 44. Therefore, anchored implantation of the stem 43 can be carried out quite quickly by the surgeon. Thereafter, the head 45 can be coupled non-rotatively with the implanted stem, in the manner described below.

Thus, the head 45 and the stem 43 are provided with interfitting components which enable the head 45 to be coupled non-rotatively with the stem 43, after implantation of the stem, so that the stem 43 projects non-perpendicularly of the planar seating face 47 of the head 45 while the head can be seated on the inclined end face 48 of the first finger bone section 41.

The interfitting components comprise a socket and a stub receivable by the socket, and in the illustrated embodiment this takes the form of a multi-sided socket 49 formed in the free end of the stem 43, and an elongate projection in the form of a multi-sided stub 50, which projects non-centrally and at a non-perpendicular angle from the planar seating face 47 of the head 45.

The illustrated profile of socket 49 and stub 50 is hexagonal, and this provides six different angular positions of adjustment of the head 45 about the general axis of the stem 43, to provide the best possible seating of the head 45 on the inclined end face 48 of the finger bone section 41. Also, the profile of the socket 49 enables the stem 43 to be screwed into positions by use of an Allen key or the like. However, if it is required to permit relative rotation, then the stub projection 50 and socket 49 may be of matching circular cross-section (not shown).

The stub 50 is slidably received by the socket 49, and this can permit a certain amount of relative longitudinal displacement (if desired), to facilitate the flexing of the two bone sections 41 and 42 after implantation.

The prosthetic joint shown in Figure 1 is an unconnected joint and which is completed by provision of a prosthetic socket section which fits in the second finger bone section 42 and is designated generally by reference 51, and which has a stem 52 and a cup 53 provided with a curved engaging surface 54 which can slide on the external surface 46 of the head 45 of the prosthetic finger joint 40.

Although not shown, acrylic or other suitable bone cements may be used in order to anchor the various components of the prosthetic joint in position after implantation.

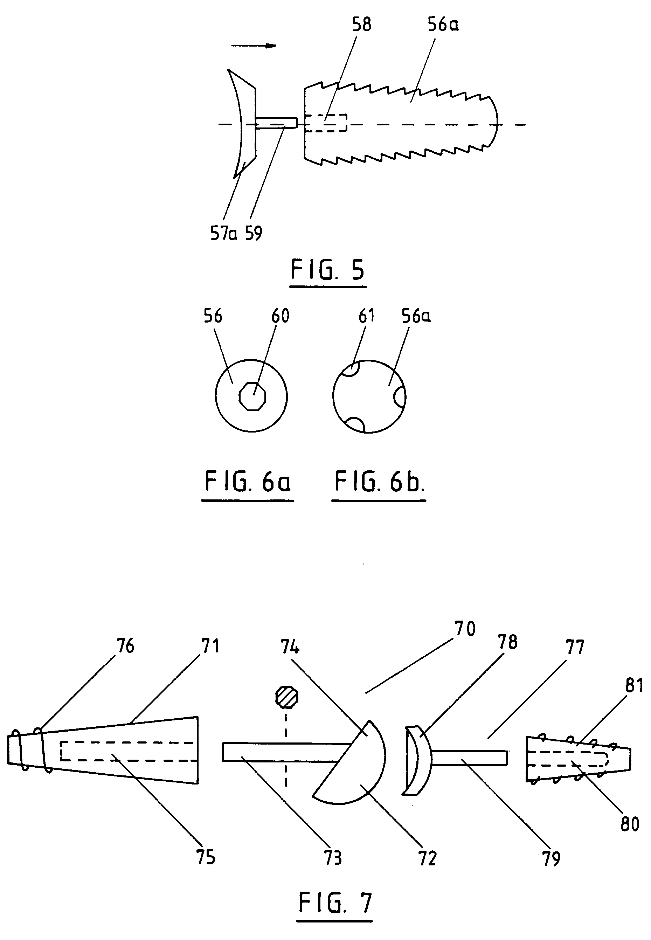

Figure 1 shows schematically the prosthetic socket section 51, and a more detailed illustration is shown in Figure 4, which is designated generally by reference 55. The device 55 comprises an externally screw threaded stem portion 56 which is tapered so that it can be introduced into position into a suitably tapering bone passage in the second finger bone section by a push fitting operation, and then anchored in position by rotation of the stem portion 56. There is also a cup shaped portion 57 which can be formed in one piece with the stem 56, or is permanently secured thereto prior to implantation (as shown in Figure 4), or which may be supplied as a separate component which is subsequently coupled with a screw threaded stem 56a, as shown in Figure 5.

The screw threaded stem 56a and cup shaped portion 57a are slidably engaged via interfitting components comprising a socket 58 and stub 59. This may provide a "floating" type of assembly, in that the cup shaped portion 57a can slide in and out during flexing of the joint.

The screw threaded stem portion 56 or 56a has a socket 60, which in the form illustrated in Figures 4 and 6 is hexagonal, and this enables the stem portion to be screwed firmly into position by use of an Allen key or the like. An alternative construction is shown in Figure 6b, in which notches 61 are provided in the stem portion 56a, to permit the required turning of the screw threaded stem 56a.

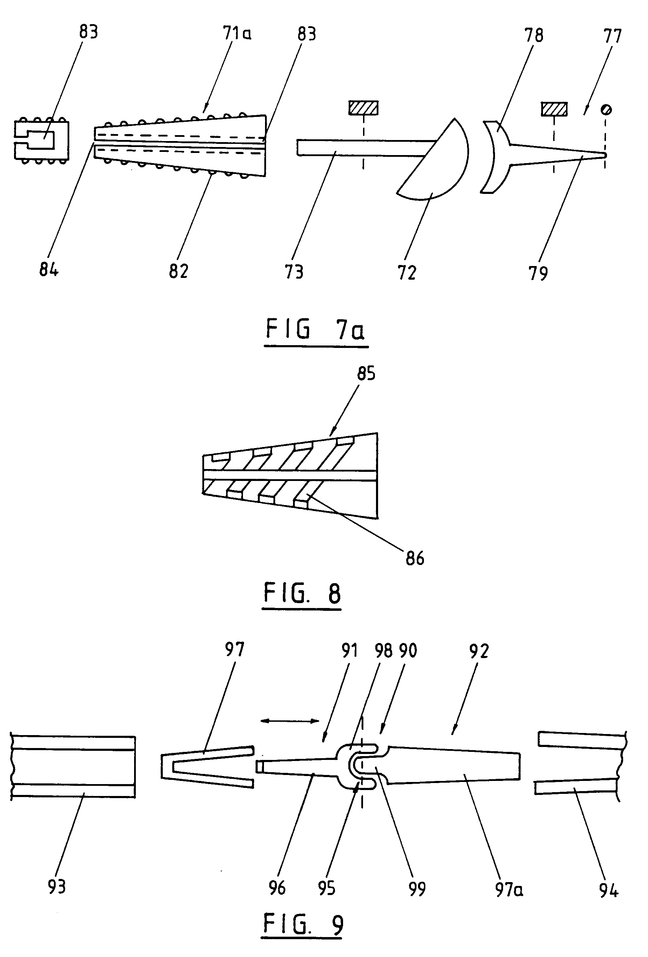

The constructions of prosthetic finger joint described above with reference to Figures 1 to 6 have been included for illustrative purposes to explain the underlying features of the invention, and preferred and more detailed examples within the scope of the invention will now be described with reference to Figures 7 to 13 of the drawings.

Referring first to Figure 7 of the drawings, this shows a prosthetic finger joint which is generally similar to that shown in Figure 1, and also is an unconnected prosthetic finger joint, in that there is no direct mechanical connection between the two basic components of the joint which are received one in each of the two adjoining finger bone sections.

The prosthetic joint shown in Figure 7 is designated generally by reference 70 and comprises an implantable stem portion in the form of a sleeve 71 which can then be introduced into a passage formed in a first finger bone section, and a head portion 72 which is engageable with an end face of the first finger section in similar manner to that described above with reference to Figure 1. An elongage projection 73 extends non-perpendicularly away from the seating face 74 of the head 72, and is of hexagonal cross sect ion as shown in Figure 7, and can be received slidably by a correspondingly shaped passage 75 formed in the stem-forming sleeve 71. Thus, once the projection 73 is received by the passage 75 of the sleeve 71, the sleeve 71 and projection 73 together form the stem portion of the prosthetic finger joint.

The sleeve 71 is externally threaded, as shown schematically by reference 76, and this can enable the sleeve to be press-fitted into position, to an anchored position, and which anchoring may be further secured by applying small rotation to the sleeve.

The prosthetic finger joint 70 also includes an implantable component which is receivable by the second bone section and cooperates with the head 72, this implantable component being designated generally by reference 77 and comprising a bearing cup 78 engageable with the outer surface of head 72, and a stem 79 projecting from the cup 78 and receivable by a passage 80 formed in a sleeve 81 which can be anchored in a suitably formed bone passage in the second finger bone section.

The prosthetic finger joint 70 shown in Figure 7 is an unconnected joint, in that there is no direct mechanical connection between the two separate components, and reliance will therefore be placed upon the existing natural tendon tissue, or a prosthetic ligament may be applied in the region of the joint after implantation.

Figure 7a shows a generally similar arrangement to that described above with reference to Figure 7, but in which projection 73 has a rectangular cross section, as also has stem 79. Further, the sleeve 71 of Figure 7 is modified to take the form of sleeve 71a shown in Figure 7a, and which has pimples or dimples 82 on its outer periphery, which enable the sleeve 71a to be press-fitted into a suitably anchored position within the finger bone section, without any necessity for tightening by rotation. The passage 83 is able to receive the projection 73 non-rotatively, but in such a way as to permit relative axial sliding movement, and sleeve 71a also has a longitudinal slit 84 in its outer periphery which allows the sleeve to expand radially outwardly upon introduction of a slightly oversized elongate projection 73, to further improve the anchoring in position.

A further alternative construction of stem-receiving or stem-forming sleeve is shown in Figure 8, and is designated generally by reference 85. Sleeve 85 is formed with a spiral groove 86 in its outer surface, into which can be laid a strip of velour or other suitable implantable fabric to promote tissue ingrowth. This could promote the anchoring in position of the sleeve over a period of time.

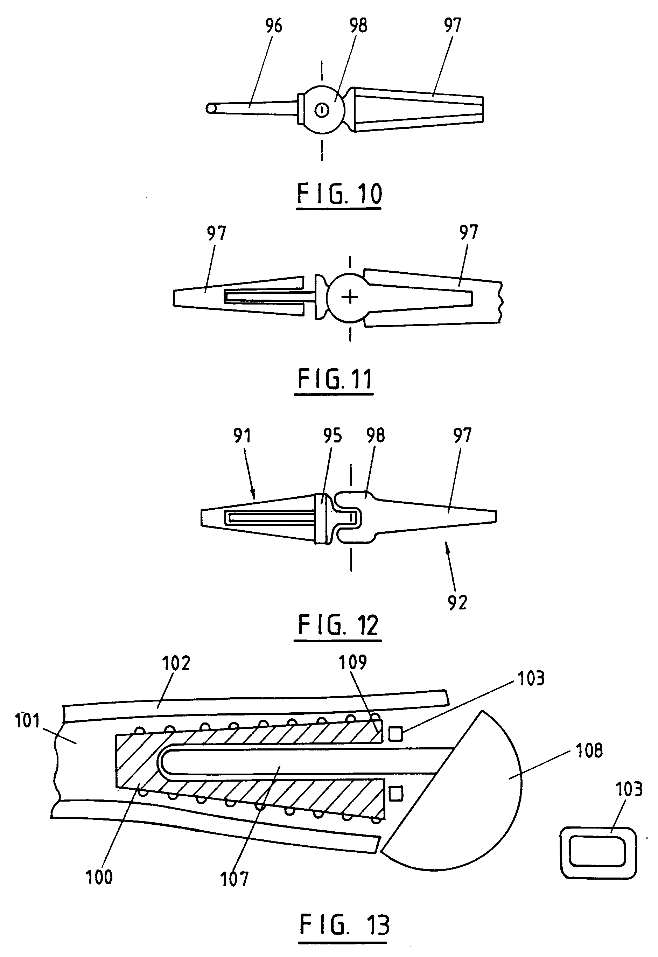

Referring now to Figure 13, this shows one half of an unconnected prosthetic finger joint, generally similar to that described thus far, and which will normally be implanted in the metacarpal component of a natural finger joint. To control the depth of insertion of projection 107 of head 108 into a passage 109 of sleeve 100 (received by passage 101 of the metacarpal component 102), a set of washers is provided, one of which is shown by reference 103 in Figure 13. This is located around the projection 107, and controls the depth of introduction of the projection 107, thereby to control tension in the joint. However, it should be understood that the spacer washers may be mounted in similar manner on the "projection" of the other embodiments of the invention described and illustrated herein.

Figure 13 is a schematic illustration only, and in practice the head 108 will seat on the inclined end face of the component 102, and the space shown in Figure 13 between washer 103 and the inclined face of the head 108 may be taken up by a wedge shaped spacer (not shown) which can be formed with the head 108 or projection 107, or which may be slidably mounted on projection 107.

Finally, reference will now be made to embodiments of prosthetic finger joint which are shown in Figures 9 to 12, and which show two different constructions of connected prosthetic finger joint.

Referring first to Figure 9, this is an exploded view of a "connected" type prosthetic finger joint which is designated generally by reference 90, and which comprises two components 91 and 92 which are implantable respectively in metacarpal component 93 and phalangeal component 94, and which are joined together via a hinged connection 95. Component 91 has a projection or stem 96 received by stem-receiving sleeve 97, (which is press-fitted into component 93) and component 92 comprises a sleeve 97a receivable by component 94.

The hinge connection 95 comprises a yoke 98 which forms a head portion of component 91, and between the limbs of which is received a tongue 99 of component 92. A suitable pivot pin (not shown) pivotally connects the tongue 99 to the yoke 98. Preferably, a limited degree of lateral movement along the axis of the pivot is permitted by a suitably loose design of the hinge connection. Although not shown, one of the components 91 or 92 will be received non-rotatably in its respective bone section, and the other will be arranged to be rotatable. This will then permit any necessary small amount of relative rotation to take place between the two bone sections during pivoting of the finger joint.

Figure 10 is a detailed side view of the finger joint 90 shown in Figure 9.

Referring now to Figures 11 and 12, this shows a generally similar arrangement of connected prosthetic finger joint, but in which the yoke 98 is provided on the implantable component 92 to be received by the phalangeal component 94, whereas the tongue 99 is provided on the implantable component 91.

## Claims

1. A prosthetic finger joint (40) for implantation between two finger bone sections (41, 42) and which comprises: an implantable stem portion (43) for introduction into a passage (44) formed in a first of the finger bone sections (41), and a head portion (45) which is engageable with an end face (48) of the first finger bone section (41) and which serves to permit pivoting of a second of the finger bone sections (42) relative to the first finger bone section; in which the stem portion (43) and the head portion (45) are formed as separate components which are capable of being coupled together after implantation of the stem portion (43), said stem portion (43) being shaped externally so as to be capable of being implanted in its respective bone passage (44) and to be anchorable therein, and said head portion (45) and said stem portion (43) being provided with interfitting components (49, 50) which enable the head portion to be slidably assembled with the stem portion after implantation of the stem portion in its bone passage, and which comprise an elongate projection (50) provided on one of the portions (45, 43) and a socket (49) in the other of the portions (43, 45) to receive the projection (50) in such a way that the projection (50) is axially slidable in the socket (40);
characterised in that the head portion comprises a hemispherical head (45) having a substantially planar seating face (47) from which said projection (50) extends non-perpendicularly.

2. A prosthetic finger joint according to Claim 1, characterised in that the projection (50) is non-rotatable within said socket (49).

3. A prosthetic finger joint according to Claim 2, characterised by an implantable socket device (5) receivable by the second bone section (42) and slidably engageable with the hemispherical head (45) to permit relative pivoting between the first and second bone sections (41, 42).

4. A prosthetic finger joint according to Claim 1, characterised in that the projection (50) is axially slidable in the socket (49) in such a way that relative rotation between the projection (50) and the socket (49) is permitted (not shown).

5. A prosthetic finger joint according to Claim 4, characterised in that the stem portion comprises a sleeve (43, 71) having a passage (49, 75) therein which forms said socket (49).

6. A prosthetic finger joint according to Claim 4 or 5, characterised by an implantable component (51, 77) receivable by the second bone section (42) and co-operable with the head portion (45) to form an unconnected joint.

7. A prosthetic finger joint according to Claim 4 or 5, characterised by an implantable component (92) receivable by the second bone section (94) and co-operable via a hinged connection with the head portion (95, 98) of the component (91) which is receivable by the first bone section (93).

8. A prosthetic finger joint according to Claim 7, characterised in that the hinged connection comprises a yoke (98) and a tongue (99) pivotally mounted between the limbs of the yoke.

9. A prosthetic finger joint according to Claim 8, characterised in that the yoke (98) is joined to the head portion (95) and the tongue (99) is joined to the implantable component (92).

10. A prosthetic finger joint according to Claim 8, characterised in that the yoke (98) is joined to the implantable component (92) and the tongue (99) is joined to the head portion (95).

11. A prosthetic finger joint according to any one of Claims 7 to 10, characterised in that the hinged connection is a loose connection to permit abduction / adduction movement.

12. A prosthetic finger joint according to any one of Claims 1 to 11, characterised by spacer washers (103) positionable on the projection (107) adjacent to the head portion (108) to vary the tension of the joint and the depth of insertion of the projection (107) into the socket (109).

13. A prosthetic finger joint according to any one of Claims 1 to 12, characterised in that the components of the joint are made of implantable grade plastics or metal.

## Patentansprüche

1. Fingergelenkprothese (40) zur Implantation zwischen zwei Fingerknochen-Abschnitten (41, 42) mit einem implantiorbaren Schaftbereich (43) zum Einfüren in einen Kanal (44), der in einen ersten der Fingerknochen-Abschnitte (41) ausgebildet ist, und mit einem Kopfbereich (45), der in Anlage mit einer Endfläche (48) des ersten Fingerknochen-Abschnitte (41) gebracht werden kann und der dazu dient, eine verschwenkung eines zweiten der Fingerknochon-Abschnitte (42) gegenüber dem ersten Fingerknochen-Abschnitt zu ermöglichen, wobei der Schaftbereich (43) und der Kopfbereich (45) als getrennte Komponenten ausgebildet sind, die nach der Implantation des Schaftbereiches (43) zusammengekoppelt werden können, und der Schalthereich (43) außen so geformt ist, daß er in seinen entsprechenden Knochenkanal (44) implantiert und in diesem verankert werden kann und wobei der Kopfbereich (45) und der Schaftbereich (43) mit wechselseitig zusammenpassenden Komponenten (49, 50) versehen sind, welche zulassen, daß der Kopfbereich nach der Implantation des Schaftbereiches in seinen Knochenkanal verschiebbar mit dem Schaftbereich zusammengebaut werden kann, und welche einen langlichen vorsprung (50) an einem der Bereiche (45, 43) und einen Sockel (49) im anderen Bereich (43, 45) umfassen, welcher den Vorsprung (50) derart aufnimmt, daß der Vorsprung (50) axial in dem Sockel (40) verschichbar ist:
dadurch gekennzeichnet, daß der Kopfbereich einen halbkugeligen Kopf (45) mit einer im wesentlichen ebenen Sitzfläche (47) umfaßt, aus welcher der Vorsprung (50) sich unter einem von 90° abweichenden Winkel wegerstreckt.

2. Fingergelenkprothese nach Anspruch 1, dadurch gekennzeichnet, daß der Vorsprung (50) Innerhalb des Sockels (49) nicht verdrehbar ist.

3. Fingergelenkprothese nach Anspruch 2, gekennzeichnet durch eine implantierbare Sockeleinrichtung (5), die von dem zweiten Knochenabschnitt (42) aufgenommen und in gleitende Anlage mit dem halbkugeligen kopf (45) gebracht werden kann, wodurch eine Relativverschwenkung zwischen dem ersten und dem zweiten Knochenabschnitt (41, 42) ermöglicht wird.

4. Fingergelenkprothese nach Anspruch 1, dadurch gekennzeichnet, daß der Vorsprung (50) in dem Sockel (49) derart axial verschiebbar ist, daß eine Relativverdrehung zwischen dem Vorsprung (50) und dem Sockel (49) möglich ist (nicht gezeigt).

5. Fingergelenkprothese nach Anspruch 4, dadurch gekennzeichnet, daß der Schaftbereich eine Hülse (43, 71) umfaßt, in der sich ein Kanal (49, 75) befindet, welcher den Sockel (49) bildet.

6. Fingergelenkprothese nach Anspruch 4 oder 5, gekennzeichnet durch eine implantierbare Komponente (51, 77), die von dem zweiten knochenabschnitt (42) aufgenommen werden kann und mit dem Kopfbereich (45) so zusammenwirkt, daß ein unverbundenes Gelenk gebildet wird.

7. Fingergelenkprothese nach Anspruch 4 oder 5, gekennzeichnet durch eine implantierbare Komponente (92), die von dem zweiten Knochenabschnitt (94) aufgenommen werden kann und mit dem Kopfbereich (95, 98) der Komponente (91), die von dem ersten knochenabschnitt (93) aufgenommen werden kann, über eine Scharnierverbindung zusammenwirkt.

8. Fingergelenkprothese nach Anspruch 7, dadurch gekennzeichnet, daß die Schernierverbindung ein Joch (98) und eine Zunge (99) umfaßt, die verschwenkbar zwischen den Schenkeln des Jochs montiert ist.

9. Fingergelenkprothese nach Anspruch 8, dadurch gekennzeichnet, daß das Joch (98) mit dem Kopfbereich (95) und die Zunge (99) mit der implantierbaren Komponente (92) verbunden ist.

10. Fingergelenkprothese nach Anspruch 8, dadurch gekennzeichnet, daß das Joch (98) mit der implantierbaren Komponente (92) und die Zunge (99) mit dem Kopfbereich (95) verbunden ist.

11. Fingergelenprothese nach einem dar Ansprüche 7 bis 10, dadurch gekennzeichnet, daß die Scharnierverbindung eine lose Verbindung ist, welche eine Abduktions-/Adduktionsbewegung ermöglicht.

12. Fingergelenkprothese nach einem der Anspüche 1 bis 11, gekennzeichnet durch Abstands-Beilagscheiben (103), die auf dem Vorsprung (107) in der Nähe des Kopfbereiches (108) positioniert werden können, wodurch die Spannung des Gelenkes und die Eindringtiefe des Vorsprunges (107) in den Sockel (109) variiert werden können.

13. Fingergelenkprothese nach einem der Ansprüche 1 bis 12, dadurch gekennzeichnet, daß die Komponenten des Colenks aus implantierbarem Kunststoff oder Metall hergestellt sind.

## Revendications

1. Prothèse d'articulation du doigt (40) destinée à être implantée entre deux sections d'os du doigt (41, 42) et comprenant : une partie tige implantable (43) destinée a être introduite dans un passage (44) formé dans une première des sections d'os du doigt (41), et une partie formant tête (45) qui peut se mettre en prise avec une face d'extrémité (48) de la première section d'os du doigt (41) et qui permet un pivotement d'une deuxième des sections d'os du doigt (42) par rapport à la première section d'os du doigt; dans laquelle la partie formant tige (43) et la partie formant tête (45) sont formées en tant que composants séparés pouvant être accouplés l'un à l'autre après implantation de la partie formant tige (43), ladite partie formant tige (43) étant formée extérieurement de manière à pouvoir être implantée dans son passage d'os respectif (44) et y être ancrée, et ladite partie formant tête (45) et ladite partie formant tige (43) étant munies de composants d'emboîtement (49, 50) qui permettent à la partie formant tête d'être assemblée de manière coulissante à la partie formant tige après implantation de la partie formant tige dans son passage d'os, et qui comprend une saillie allongée (50) disposée sur l'une des parties (45, 43) et une fiche (49) dans l'autre des parties (43, 45) pour recevoir la saillie (50) de telle manière que la saillie (50) peut coulisser axialement dans la fiche (40) ;
caractérisée en ce que la partie formant tête comprend une tête hémisphérique (45) ayant une face d'appui (47) substantiellement plane, depuis laquelle ladite saillie (50) s'étend non-perpendiculiarement

2. Prothèse d'articulation du doigt selon la revendication 1, caractérisée en ce que la saillie (50) ne peut pas tourner a l'intérieur de ladite fiche (49).

3. Prothèse d'articulation du doigt selon la revendication 2, caractérisée par un dispositif à fiche implantable (5) pouvant se loger dans la deuxième section d'os (42) et se mettre en prise de manière coulissante avec la tête hémisphérique (45) pour permettre un pivotement relatif entre les première et deuxième sections d'os (41, 42).

4. Prothèse d'articulation du doigt selon la revendication 1, caractérisée en ce que la saillie (50) peut coulisser axialement dans la fiche (49) de telle manière qu'une rotation relative entre la saillie (50) et la fiche (49) est permise (non représenté).

5. Prothèse d'articualtion du doigt selon la revendication 4, caractérisée en ce que la partie formant tige comprend une douille (43, 71) comportant un passage (49, 75) formant ladite fiche (49).

6. Prothèse d'articulation du doigt selon la revendication 4 ou 5, caractérisée par un composant implantable (51, 77) pouvant se loger dans la deuxième section d'os (42) et coopérant avec la partie formant tête (45) pour former une articulation non-liée.

7. Prothèse d'articulation du doigt selon la revendication 4 ou 5, caractérisée par un composant implantable (92) pouvant se loger dans la deuxième section d'os (94) et coopérant via une liaison articulée avec la partie formant tête (95, 98) du composant (91) qui peut se loger dans la première section d'os (93).

8. Prothèse d'articulation du doigt selon la revendication 7, caractérisée en ce que la liaison articulée comprend un étrier (98) et une languette (99) montée pivotante entre les branches de l'étrier.

9. Prothèse d'articulation du doigt selon la revendication 8, caractérisée en ce que l'etrier (98) est raccordé à la partie formant tête (95) et la languette (99) est raccordée au composant implantable (92).

10. Prothèse d'articulation du doigt selon la revendication 8, caractérisée en ce que l'étrier (98) est raccordé au composant implantable (92) et la languette (99) est raccordée à la partie formant tête (95).

11. Prothèse d'articulation du doigt selon l'une quelconque des revendications 7 a 10, caractérisée en ce que la liaison articulée est une liaison lâche pour permettre un mouvement d'abduction/adduction.

12. Prothèse d'articulation du doigt selon l'une quelconque des revendications 1 a 11, caractérisée par des rondelles d'écartement (103) pouvant être placées sur la saillie (107) à proximité de la partie formant tête (108) pour varier la tension de l'articulation et la profondeur d'insertion de la saillie (107) dans la fiche (109).

13. Prothèse d'articulation du doigt selon l'une quelconque des revendications 1 à 12, caractérisée en ce que les composants de l'articulation sont fabriqués en plastique ou metal de qualité implantable.
